# EUROPEAN PATENT SPECIFICATION

(11) **EP 3 445 286 B1**
(45) Date of publication and mention of the grant of the patent: **28.12.2022**
(21) Application number: 17734148.4
(22) Date of filing: 21.04.2017
(51) Int. Cl.: A61F 2/12, A61F 2/00

(54) **MEDICAL DEVICE FOR BREAST RECONSTRUCTION TO AVOID THE PTOSIS AFTER PROSTHESYS IMPLANT**
MEDIZINPRODUKT FÜR DIE BRUSTREKONSTRUKTION ZUR VERMEIDUNG VON HÄNGEBRUST NACH DER IMPLANTATION EINER PROTHESE
DISPOSITIF MÉDICAL POUR RECONSTRUCTION MAMMAIRE, CONÇU AFIN D'ÉVITER LA PTOSE APRÈS IMPLANT DE LA PROTHÈSE

(30) Priority: 21.04.2016 IT UA20163329
(43) Date of publication of application: 27.02.2019
(73) Proprietor: DECO MED SRL, 30020 Marcon (VE) (IT)
(72) Inventor: BERTOLI, Giovanni, 31021 Mogliano Veneto (TV) (IT)
(86) International application number: PCT/IT2017/000082
(87) International publication number: WO 2017/183055

(56) References cited:
- WO-A1-2006/117622
- WO-A1-2014/041577
- US-A1- 2012 053 690
- US-A1- 2014 088 700
- US-A1- 2015 351 891

## Description

The present invention , defined in claim 1, relates to a medical device for breast reconstruction that helps to prevent the ptosis of tissues due to sagging under the weight of a breast implant.

Nowadays it is known the need to provide for a breast reconstruction after mastectomy.

The current surgical techniques of breast reconstruction after mastectomy )is divided into two techniques: one in two times and one in a single time.

The technique in two stages provides that, completed the mastectomy, the surgeon prepares a submuscular pocket creating a space between the chest wall and the pectoralis major muscle, which is low-cut and etched down to the superficial fascia to its lower pole and medial until the fourth 15space intercostal.

Laterally it then proceeds to the incision of the serratus muscle fascia. Then it places a specific tissue expander and closes the pocket in such a way that it includes, at its inside all the coverage.

In the following days there shall be a progressive inflation until obtaining the desired expansion.

Then the expander is removed, not before 4/6 months, with a second operation that will include the removal of the expander and the placement of the final prosthesis.

In the technique in a single time, the reconstruction involves the placement, after mastectomy, of a breast implant in a under-muscular pocket, created with the technique described previously.

The breast implant, subsequently inserted into the under-muscle cavity, is covered at its lower pole with a mesh of biomaterial, thereby avoiding the need for a muscle expansion.

The results of the two techniques are similar for small and medium sized breasts.

The large breasts currently represent an indication for reconstruction in two stages.

The results of the two techniques are similar from a physiological point of view, because, in any case, there is the resection and the dissection of the pectoralis major muscle that entails, its dislocation and therefore a substantial loss of functionality.

In relation to the physiology of the pectoralis major muscle, in fact, it can be stated that in case of disconnection of it, the movements that may be deficient, are the anteposition and flexion of the arm, the internal rotation and adduction.

In addition, considering the functionality of the shoulder and upper limb, it is necessary to assess the synergistic action of the modulated various muscle groups; also the weakening of quantitatively modest part can alter the shoulder-joint and affect the normal course of activities of daily living. In fact, the pectoralis major muscle is located on the chest, anteriorly, between the medial part of the clavicle, sternum, cartilages of the first 6/7 coasts, the external oblique aponeurosis (medially) and the crest under the greater tuberosity of the humerus (sideways).

It is divided into two parts: the head and clavicular head sternocostal, which differ in their effects on motility of the shoulder and upper limb. The clavicular head flexes the arm, it alleges toward the contralateral shoulder and rotates it internally.

The sternocostal head depresses the shoulder, adducts the arm towards the contralateral iliac crest and rotates internally.

These movements are combined in various ways, each other and with the movements generated by many other muscles acting on the shoulder, in giving life to normal gestures.

The shoulder, both anatomically and functionally, is an extremely complex mechanism that has a very wide motor ability.

This entails the need to dispose of activation synergies, adjustment and balancing of different muscular components in relation to the control of multiple engine parameters (direction, distance, strength, speed, endurance) to produce a qualitatively "normal movement", that is accurate, fluid, suitable for the motor task requested.

The movements which may result insufficient, in case of partial lesion of the pectoralis major, are the anteposition and flexion of the arm, the internal rotation and adduction, in more or less evident manner, in relation to the position and to the extent of the injury .

In addition, in relation to the functionality of the shoulder and upper limb, we must consider that the synergistic action of the modulated various muscle groups, also the weakening of a part quantitatively modest, can alter the sholder-joint with repercussions on the normal course of everyday life.

In the state of the art the advantages described have been overcome with the invention of patent IT278047.

The object of the patent application IT278047 is constituted by a flat element, divided into several partitions, which define a central planar area with first bases slightly arched. The baffles are made so that they can be folded by combining the respective first sides adjacent which, once joined together by, for example, sutures, define a containment structure for a breast implant, forming a box-like element adapted to contain, containing or integral a breast implant, said box-like element, being interposed between the skin and the pectoralis major muscle and being suturabile to the latter.

In relation to the above-mentioned patent, it should be noted how the geometric figure, object of the invention, allows a complete winding of a mammary endoprosthesis in order to allow a better acceptance from the body, because the bio-active biological matrix (patent n. IT278047) is interposed between the silicon (biomaterial inert) and vital tissues.

Said solution enables the breast reconstruction on the supra-pectoral muscle without the need to detach the pectoralis major muscle because the biomaterial bio-active of which is made the device (n.IT278047 patent), interferes with the onset of capsular fibrosis, even if the prosthetic implant is positioned on the subcutaneous plan inside the human body.

Said patent becomes, in fact, autologous tissue that isolates the silicone implant from the context of tissue and, in fact, makes it better accepted from the body.

This neo-autologous tissue has been evaluated clinically as a thin layer of neo-well vascularized fascia. From the histological point of view, is constituted by typical cells of the fascial tissue (fibroblasts, myofibroblasts).

After implantation in the human body, the silicone implants wrapped by such neo-tissue (derived from the surgical implant of the device of patent IT278047 patent) weighs down on the lower pole of the breast, because the neo-tissue which envelopes the prosthesis, behaves like all human tissues: under a weight, it yields.

Such subsidence of breast skin mantle, is defined ptosis and it is measured through a scale where with PAR 1 is defined as the subsidence of 1 cm with respect to the furrow under-breast, with PAR 2 two cm with respect to the furrow under-breast and PAR 3 three cm with respect to the furrow under-breast.

The invention, object of the patent cited above, today's state of the art, it is already an overrun of devices referred to state of the art. These devices, in fact, do not allow the full winding of the prosthesis and thus the breast implant placement on the floor under-cutaneous (or supramuscular), and so there is the risk of capsular fibrosis.

Nevertheless said patent, like all the other devices of the state of the art (in breast reconstruction with implants), it does not solve the problem of control of breast ptosis.

As said, the ptosis is due to the subsidence of breast skin - mantle, due to loss of elasticity of the skin mainly at the inferior pole of the breast under the weight of the mammary gland.

The physiological wiught on the lower pole is not equal to the weight of the mammary gland as an anatomic piece, but is lower and it is how allowed from the anatomical structure of the breast. The gland is integral with the pectoralis major-muscle wall thanks to the suspensory ligament of the breast and with the subcutis thanks to the suspensory ligaments of Cooper. These ligaments discharge the weight of the gland on the said structures and allow the transfer only in part, of the weight of the the mammary gland overall,in the inferior pole.

The weight of an anatomical, glandular part, normally varies between 100 and 800 grams, corresponding to a first or sixth bra size or more.

The weight exerted on the lower pole of the breast in the anatomicalphysiological normal conditions (non-mastectomy patient), however, is not of the weight of the anatomical part. As said, anatomically the gland is integral with the muscular wall and the sub-cutaneous tissue using the suspensory ligaments that subtract weight to the lower pole. In anatomonormal physiological conditions, the inferior pole of the breast is burdened with a less weight than the weight of the mammary gland considered as anatomical part.

The implantation of a breast implant of weight comparable to an anatomical part, missing the suspensory ligaments because of mastectomy, alters the physiological weight on the lower pole of the breast, burdening it of an abnormal weight.

The patient's proprioceptive perception, will be different than before the implant, because she feels a weight at the lower pole that was not there before.

On the other side the breast implants for reconstruction post-mastectomy are constituted by silicone envelope, containing cohesive silicone gel. Most of Companies codify silicone implants for volume (cubic centimeters) that corresponds, more or less, to a weight of equal grams, increased by ten percent.

The choice of size of the breast implant to be implanted depends on the surgeon that normally evaluete the subtracted quantity(ill gland) to restore it with a silicone implant of equal volume.

If the surgeon, however, chooses for a breast implant size such as to restore the physiological weight on the lower pole of the breast, should implant a volume-size inferior than a size useful to harmonize with the contralateral breast. If the surgeon did it, should reconstruct a disharmonious breast (in relation to the volume) compared to the contralateral breast which is healthy. Therefore, the surgeon usually choose for a prosthesis volume equal to the glandular explanted with the eye to the harmonic symmetry with the volume of the contralateral breast.

The mammary prosthesis choosen, will exercise on the inferior pole of the breast an abnormal weight compared to the physiological weight, which is yielded from the gland holded to the tissue by suspensory ligaments.

The "dead"-weight of the prosthesis, deprived of the suspensory ligaments, will weigh on tissues, including the neo-tissue (the aforementioned patent) of the lower pole, favoring in time the development of ptosis (PAR).

It is believed, in summary, that the invention of the aforementioned patent, does not allow the control of ptosis because, after being transformed into a thin vascularized tissue, can yield under "dead"- weight of a breast implant that is not anchored to muscular wall and at the subcutis like it was the gland, thanks to the suspensory ligaments.

In the state of the art there are other patents which, however, claiming different methods and procedures.

The patent no. US2015 / 351 891 refers to a system for mastopexy which is formed in a different way by the invention. In fact it presents two elements: "suspension strut" that the invention does not claim, and that which acts as "attachment" to the second pole of the lower support element. This second element is shaped in canoeing, different from the invention. Also, the material is different. In par. 62 In fact, the patent US 2015/351891 speaks about synthetic material which can be used alone or "in blends" with another biomaterial. Our invention, however, the synthetic biopassiva mesh, must always be used with the organic matrix bioactive; then they are not mixed but rather joined (the first plus the second).

Patent US2014 / 088700 is a method to use a silk scaffold.

This patent is different from our invention for: - the shape. In fact, the patent US2014 / 088 700 is presented as a trapezoid form or canoe; our invention has a well-defined and unique shape; - the material. In fact, the patent US2014 / 088 700 claims a silk device. Our invention is in biological matrix (ECM) bioactive added to a biocompatible synthetic material biopassive; -surgical tecnique in which you have to use. The patent US2014 / 088 700 is used in breast reconstruction "two steps"procedure with expander. Our invention is used in reconstructive procedure "one step" without the use of expander.

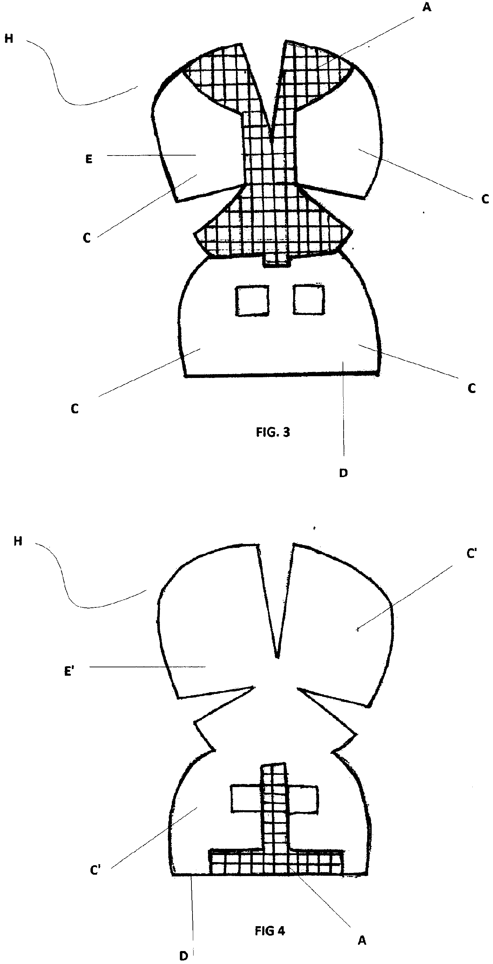

The patent US2012/05690 claims a different device by our invention. In fact, the patent US2012/05690 has a different shape compared to the invention. It presents itself as a structure that doesn't cover all the prosthesis but it is only a support in the lower pole. In addition, the patent is positioned under the muscle while the invention is placed over the pectoralis major muscle. Finally, the patent US2012/ 05690 refers in par. 55 to the synthetic network is covered with a biological matrix. Our invention has a synthetic mesh covered with a matrix but the two materials are interconnected as best seen from Table 2 Figs. 3-4. In fact, the invention may have a hole that allows the passage from the front to the rear (interconnection).

Finally The patent WO2006/117622 claims a device made exclusively of synthetic material, P4HB. The invention, however, is a combination/union of two materials: a bioactive matrix (ECM) and a synthetic mesh that intersect in the center. The patent WO2006 / 117622, also does not have a specific shape and defined like our invention. WO2014041577 A1 discloses a medical device for breast reconstruction, which is constituted by a box element obtained from a flat sheet, made from a biomaterial, divided into multiple partitions, sewn together to define work in the front, side, top and bottom of said box element.

Purpose of the invention object of this patent application is to solve the problem of the relationship between suitable volume of a breast prosthesis and its weight to the inferior pole of the breast in order to control the ptosis due to tissue failure over time and not to alter the patient's proprioceptive perception.

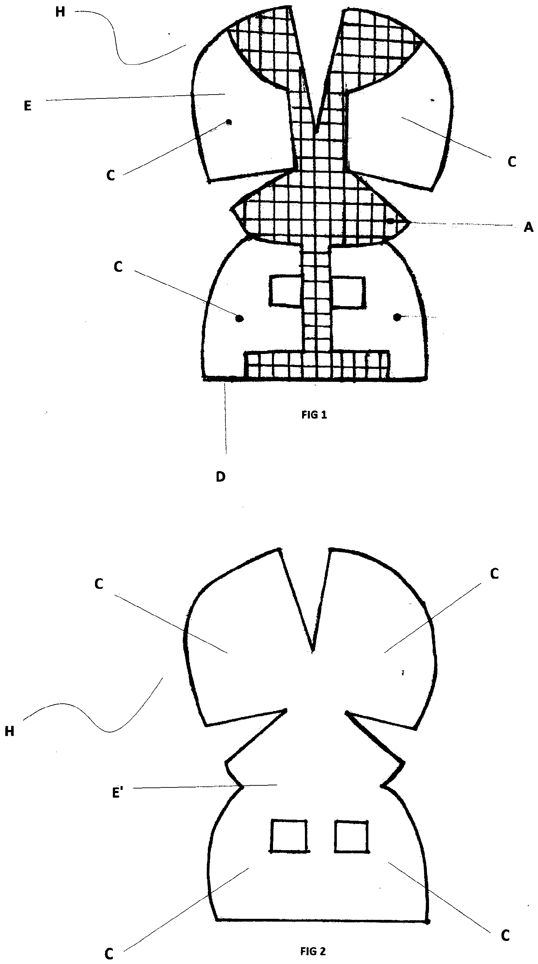

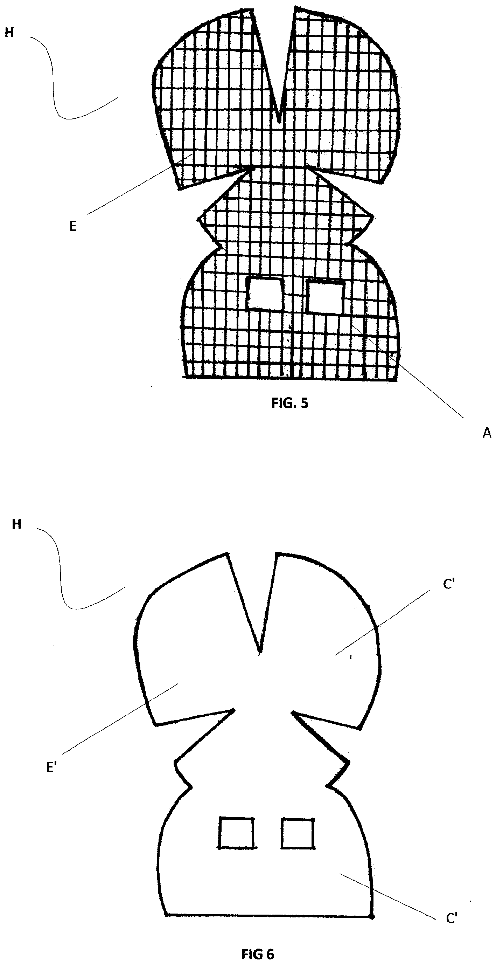

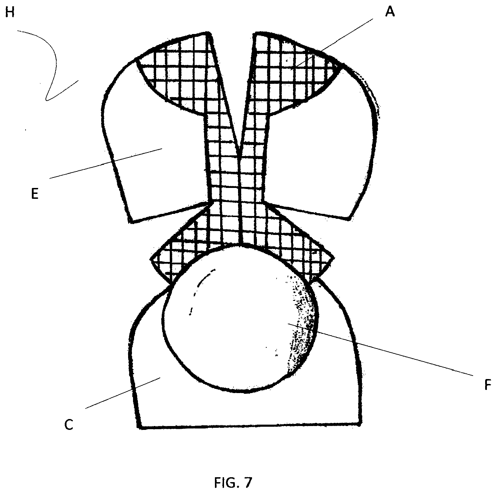

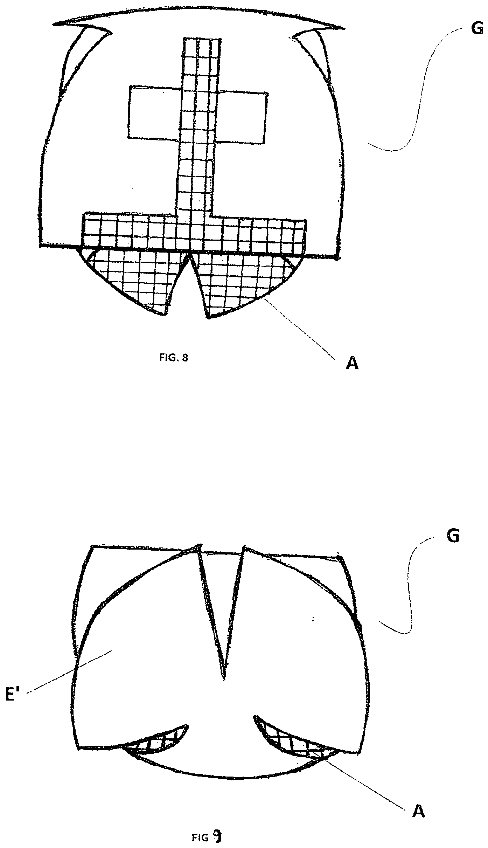

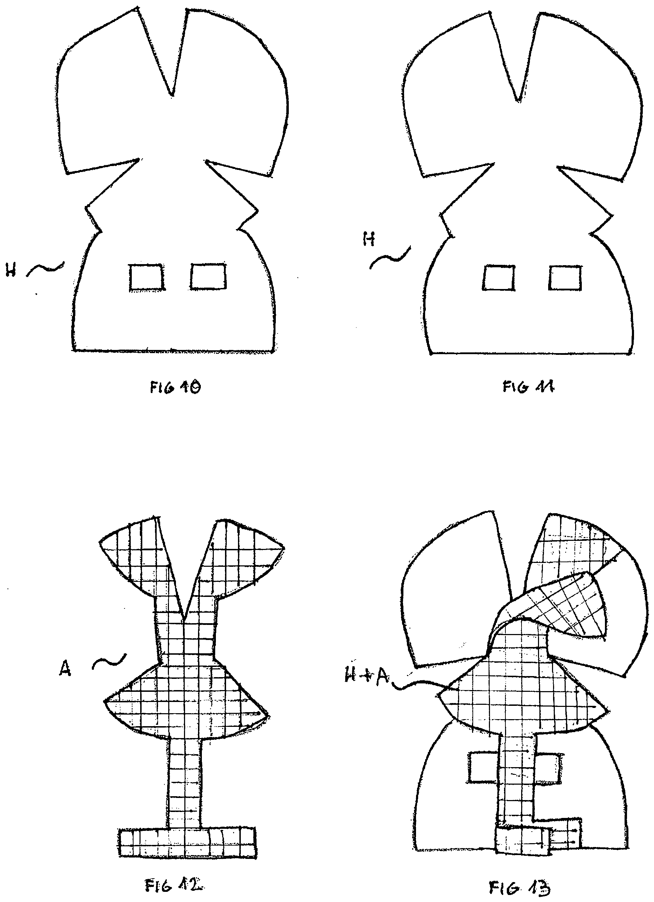

Further characteristics and advantages of the invention will become apparent from the detailed description of a particular but not exclusive embodiment, illustrated only by way of non-limitative example in the accompanying drawings, in which:
Table 1 - Fig. 1 - Fig. 2
Exemplification of a first embodiment mode;
Fig. 1 - Front view internal
Fig. 2 - Exterior Rear View
Table 2 - Fig. 3 - Fig. 4
Exemplification of a second embodiment mode;
Fig. 3 - Internal Front view
Fig. 4 - Exterior Rear View
Table 3 - Fig. 5 - Fig. 6
Exemplification of a third embodiment mode;
Fig. 5 - Internal Front view
Fig. 6 - Exterior Rear View
Table 4 -Fig. 7
Exemplification of the positioning of the prosthesis on the second embodiment mode of the invention
Table 5 -Fig. 8 - Fig. 9
Exemplification of the invention in the second embodiment mode wrapped on the prosthesis
Fig 8 - found on the prosthesis assembled rear view
Fig 9 - found on the prosthesis assembled front view
Table 6- Fig. 10 - Fig.11.- Fig.12 - Fig. 13
Fig. 10 and 11 The bioactice matrix (ECM) front and back view
Fig. 12 the biopassive mesh (syntetic)
Fig. 13 medical device made of bioactive matrix plus biopassive mesh.

In relation to any one of the represented embodiments, the invention disclosed in the present patent application, is constituted by a bio-passive mesh (A) in little elastic inert biomaterial applied to overlapping or interconnected to the inner surface (E) of a flat element (H) said flat element (H) being made of collagenous biomaterial and being divided into multiple partitions (C) which define a central planar area with a base (D). The baffles (C) are made so that they can be folded by combining the respective first sides adjacent which, once joined together by, for example, sutures, define a containment structure for a breast implant (F), forming a box- element (G) adapted to contain, containing or integrant a breast implant(F), said box-element (G), being interposed between the skin and the pectoralis major muscle and being suturabile to the latter, forming with it a bi-phasic device in collagenous biomaterial in which is composed the flat element (H) plus bio-passive biomaterial which is composed the mesh(A), realizing a "anti-capsule pouch", but inextensible, adapted to prevent the incongruous weight-transfer to the inferior pole of the breast given from the dead weight of the mammary prosthesis.

The system realized according to what has been described replaces the function of suspensory ligaments that in the normal anatomicalphysiological conditions (not subject to mastectomy patient) maintain high the mammary gland.

The function of the collagen material (flat element (H) with the inner surface (E) and outer surface (E)) is to isolate from the tissue-context the bio-passive materials (silicone implants and synthetic mesh) and then prevent capsular fibrosis.

The function of the invention object of the present patent application, constituted of bio-passive element, synthetic mesh (A) applied to overlapping or interconnected to the inner surface (E) of the flat element (H) in collagenous material, is to absorb most part of the weight due to the dead -weight of a breast implant replacing the function of the suspensory ligaments.

The biomaterial of the mesh (A) is bio-inert passive type because it must not be reabsorbed by the body over time, exercising his sospensoria function.

Biomaterial may be of polypropylene, polyethylene, ultra high molecular density, material Gore-tex type r, polypropylene type, Titanium and in any other non-resorbable biomaterial.

The shape of the mesh (A) (Table 1, 2, 3, 6) can change, being complementary to the flat element (H) in collagenous material, having to constitute with it a single bi-phasic structure, capable of coating a breast implant, limiting the risk of capsular fibrosis and at the same time avoiding an incongruous weight to the inferior pole of the breast.

The measurement of the lattice of the mesh (A), will preferably but not exclusively "large" to decrease the risk of constituting pabulum for bacteria and in order to achieve a deformability such as to fit the shape of the primary device in collagen. Along the peripheral edges, for a variable band around 5 or more or less cm., will be constituted by a tighter mesh to be able to be shaped with scissors, together with the primary device, keeping intact the interweaving weft-warp, and then the its mechanicalsospensoria function.

From the biological point of view, the neo-capsule will be constituted by a thin vascularized layer with presence of fibroblast cells, fibrocytes, myofibroblasts, typical of the fascial tissue, but interconnected with synthetic structure fibro- reticular, non-resorbable, configuring an enveloping, suspensory element of the prosthesis mammary.

## Claims

1. Medical device, for breast reconstruction, consisting of a flat element (H) in bioactive matrix biomaterial ECM, said flat element (H) having an inner surface (E) and being said flat element (H) divided into more septa (C), said septa (C) defining a flat central area (D) with a base and made so that they can be folded by combining the respective adjacent first sides which, once united for example with points of suture, define a box structure for a breast prosthesis (F), forming a box element (G) adapted to contain, containing or integrant, a breast implant (F), said box element (G), being interposable between the skin and the pectoralis major muscle and being suturable to the latter, **characterized in that** a biopassivesynthetic mesh (A) overlaps the inner surface (E) of said flat element (H) wherein the biopassive- synthetic mesh (A) is made of polypropylene or polyethylene and wherein the flat element (H) and the biopassive- synthetic mesh (A) constituting an assembled inextensible device.

2. Device as in claim 1 **characterized in that** the bioactive matrix is collagenous biomaterial.

3. Device according to the preceding claims which is **characterized by** the fact that the biopassive- synthetic mesh (A) has a loose net structure in the centre, while along the peripheral sides, for a variable side of about 5 cm, to allow a better internal framing, maintaining intact the interweaving weft-warp.

## Patentansprüche

1. Medizinprodukt für die Brustrekonstruktion, bestehend aus einem flachen Element (H) aus einer bioaktiven Matrix aus dem Biomaterial ECM, wobei das besagte flache Element (H) eine Innenfläche (E) aufweist und das besagte flache Element (H) in mehrere Septen (C) unterteilt ist, und die besagten Septen (C) einen flachen zentralen Bereich (D) mit einer Basis bilden und so gemacht sind, dass sie gefaltet werden können, indem die jeweiligen aneinandergrenzenden ersten Seiten verbunden werden, die, nachdem sie beispielsweise mit Suturstichen vereint wurden, eine Schachtelstruktur für eine Brustprothese ergeben (F), indem sie ein Schachtelelement (G) bilden, das für die Aufnahme eines Brustimplantats (F) geeignet ist, indem es letzteres aufnimmt oder integriert, wobei das besagte Schachtelelement (G), da es zwischen die Haut und den Musculus pectoralis major eingesetzt und an letzteren genäht werden kann, **dadurch gekennzeichnet ist, dass** ein biopassives Synthetik-Gittergewebe (A) die Innenfläche (E) des besagten flachen Elements (H) überlappt, wobei das biopassive Synthetik-Gittergewebe (A) aus Polypropylen oder Polyethylen gemacht ist und wobei das flache Element (H) und das biopassive Synthetik-Gittergewebe (A) ein zusammengesetztes, unausdehnbares Produkt darstellen.

2. Medizinprodukt wie in Anspruch 1, **gekennzeichnet dadurch, dass** die bioaktive Matrix aus kollagenhaltigem Biomaterial besteht.

3. Medizinprodukt gemäß den obigen Ansprüchen, **gekennzeichnet durch** die Tatsache, dass das biopassive Synthetik-Gittergewebe (A) in der Mitte eine lockere Netzstruktur aufweist, während es entlang der peripheren Seiten für eine variable Seite von ungefähr 5 cm zur Ermöglichung einer besseren internen Umrahmung die Webart mit Schuss- und Kette beibehält.

## Revendications

1. Dispositif médical, pour la reconstruction du sein, consistant en un élément plat (H) en biomatériau de matrice bioactive ECM, ledit élément plat (H) ayant une surface interne (E) et ledit élément plat (H) étant divisé en plusieurs septa (C), lesdits septa (C) définissant une zone centrale plate (D) avec une base et étant réalisés de manière à pouvoir être pliés en associant les premiers côtés adjacents respectifs qui, une fois réunis par exemple par des points de suture, constituent une structure boîte pour une prothèse mammaire (F), formant un élément boîte (G) adapté pour contenir, contenant ou intégrant, un implant mammaire (F), ledit élément boîte (G), étant interposable entre la peau et le muscle grand pectoral et pouvant être suturé à ce dernier, **caractérisé en ce qu'**une maille synthétique biopassive (A) recouvre la surface intérieure (E) dudit élément plat (H), la maille synthétique biopassive (A) étant faite de polypropylène ou de polyéthylène et l'élément plat (H) et la maille synthétique biopassive (A) constituant un dispositif inextensible assemblé.

2. Dispositif selon la revendication 1, **se caractérisant par le fait que** la matrice bioactive est un biomatériau coliagénique.

3. Dispositif selon les revendications précédentes qui est **caractérisé par le fait que** la maille synthétique biopassive (A) a une structure de filet lâche au centre, plutôt que le long des côtés périphériques, pour un côté variable d'environ 5 cm, pour permettre un meilleur encadrement interne, en maintenant intact le tissage des fils de chaîne et de trame.
